# EUROPEAN PATENT APPLICATION

(11) **EP 1 233 267 A1**
(43) Date of publication of application: **21.08.2002**
(21) Application number: 01200519.5
(22) Date of filing: 14.02.2001
(51) Int. Cl.: G01N 33/12, G01N 27/64

(54) **Method for detecting boar taint by determining skatole and androstenone concentrations**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Van Dijk, Reijer, 3906 BH Veenendaal (NL)
(74) Representative: Jorritsma, Ruurd

(57) **Abstract**

The present invention relates to a method for detecting an unpleasant odour in animal bodies by determining the skatole and androstenone contents in said bodies. Said method comprises a sample collecting step and a detection step using ion mobility spectrometry. Furthermore, the invention covers a device for carrying out the above-mentioned method.

## Description

### Field of the invention

The present invention relates to a method for detecting an unpleasant odour in animal bodies by determining the skatole and androstenone contents in said bodies.

### Background of the invention

Meat from uncastrated boars can develop a fecal- or urine-like smell, called boar taint. This bad smell is associated with poor meat tenderness and often with bitter taste of the meat. A major problem associated with boar taint is that it is not detected until the meat is heated. In contrast to uncastrated boars, boar taint rarely occurs in castrated boars. For this reason male pigs are usually castrated at a young age.

Unfortunately, the castration of male pigs has several disadvantages. Castration is associated with substantially higher production costs, since castration entails labour costs and possible animal losses. Eventually, this results in higher consumer prices. Besides that, castration leads to suffering of the animals. Furthermore, castrated boars need more feed to grow and develop more frequently a disease. A concomitant disadvantage of the increase in feed utilization of castrated boars is the increased excretion of pollutants, especially nitrogen-containing compounds, in the manure. Additionally, the quality of the meat of castrated boars is lower, since the fat-meat ratio is higher. Although the above-mentioned disadvantages of castration are manifold, the problem of boar taint is an important limitation for the use of uncastrated boars in the pork industry. A possible solution for this situation is sorting out the carcasses, which may develop boar taint, on the slaughter line and process the sorted carcasses into products where boar taint is not important. This way the meat which may develop boar taint will not reach the consumer. The above-mentioned solution can only be applied when boar taint is detectable early in the production process of meat, preferably early on the slaughter line.

Today two categories of methods are used to detect boar taint. These methods can be divided in direct and indirect methods. Direct methods comprise organoleptic methods. An example of a well known organoleptic method is the following. A piece of meat is placed in an oven at 200°C for 6-7 minutes and the taste and smell of the meat is rated by a panel of 8 persons. It is clear that organoleptic methods have several disadvantages. They are often subject to considerable variation and not practicable on the slaughter line, since they are time consuming. Indirect methods comprise chromatographical, immunological and spectrometrical methods. In these methods, the compounds responsible for boar taint in uncastrated boars play an important role. Namely, the detection of these compounds is used as a measure of boar taint. The compounds contributing to boar taint are known. Although some reports exist stating that androstenone or skatole alone are responsible for boar taint, it is generally believed that both compounds are contributors to boar taint (see Bonneau (1998) Meat Science 49, S257-S272).

Several indirect methods have been developed to detect boar taint by determining the above mentioned compounds. US4906563 and Mortensen and Sorensen (1984) 30th European Meeting of Meat Research Workers, Bristol, both describe a method for detecting boar taint comprising the determination of skatole in pigs. The former uses a method based on an immunoassay and the latter uses a spectrophotometric method. A disadvantage of both methods is that they only detect skatole, and not androstenone. A further disadvantage is that both methods make use of several steps, e.g., an extraction step with organic solvents and a filtration or incubation step before the final detection step. Therefore, these methods are laborious and expensive and not usable on a slaughter line.

Known methods to detect androstenone are pyrolysis-mass spectrometry (see Berdagué *et al*. (1996) Sciences des Aliments 16, 425-433), radioimmunoassay, thin layer chromatography, high performance liquid chromatography in combination with gas chromatography and enzyme-linked immunosorbent assay. Said methods have the disadvantage that they only detect androstenone and require sample preparation, such as extraction, separation and purification. Said preparation steps make them time consuming and expensive and thus not suitable for use in a slaughterhouse.

A method known to detect skatole as well as androstenone is disclosed in US4384206. This method is charaterized in that IR spectrophotometrical transmission data are determined for the individual carcasses and compared with corresponding data having a statistical relationship to boar taint. A disadvantage of this method is its reliability. It is therefore recommended to determine additional parameters with statistical correlation to boar taint. These additional determinations make this method slow and thus not useful for use on the slaughter line.

The object of the present invention is to provide a method which avoids the disadvantages of the above-mentioned methods and is industrially applicable, i.e. on the slaughter line, in determining boar taint.

The above-mentioned object is obtained by a method according to the preamble, characterized in that, it covers the following steps:
a) collecting a sample from an animal body and
b) determining the content of gaseous compounds in the sample by ion mobility spectrometry.

One or more of the components responsible for the unpleasant odour in animal bodies can be determined in said animal bodies by the method according to the invention. The unpleasant odour can be found in cattle, sheep, goats and pigs. It is particularly observed when heating meat from uncastrated pigs, called boars. So the unpleasant odour is mostly referred to as boar taint. The main components of boar taint, i.e. skatole and androstenone, can be detected by the above method. Preferably, other possible components of boar taint such as certain fatty acids, hexanal, androstenol, androstenan, androstenandiene, androstadienine and conversion or breakdown products thereof can also be determined in the animal bodies by the method according to the invention.

Furthermore, in the method according to the invention the sample is preferably collected directly from an individual animal body, preferably a carcass or a part thereof, but alternatively it can also be taken from any preparation of said animal body, carcass or a part thereof such as a meat or fat extract or other preparation obtained by any method known in the art suitable for the method according to the present invention.

In a preferred embodiment of the invention the collection of the sample comprises the following steps:
I) locally heating the meat surface;
II) placing a sample collection device on the heated meat surface, and
III) leading the sample in the ion mobility spectrometer.

Preferably, the heating is performed on an individual animal body but it can also be performed on the surface of a carcass or a part thereof. Since androstenone is soluble in fat, it mainly accumulates in fatty parts of the animal body such as the chine of the animal body. Therefore, the heating is preferably performed on these parts of the body In another embodiment of the invention the heating is performed on an extract or other preparation of a meat or fat sample obtained by any method known in the art suitable for the method according to the invention. When an individual animal body, a carcass or a part thereof is inspected the heating is preferably performed on the surface of said piece of meat or fat outside the ion mobility spectrometer. An extract or other preparation of an animal body may also be inserted into the ion mobility spectrometer and subjected to heating inside said spectrometer.

The method according to the invention is performed at a temperature necessary to evaporate the components of interest. Heating can be performed by lasers, preferably infrared lasers, by placing a heated object against the surface or sample or by radiation. Preferably, the heating is performed at 20-100°C, more preferably at 30-80°C and in particular at 40-70°C.

When the surface of an individual animal body, a carcass or a part thereof or an extract or other preparation thereof is heated outside the ion mobility spectrometer, an evaporated sample can be collected by any collection device capable of leading the evaporated sample into the ion mobility spectrometer. The collection device can comprise a vacuum pump or another device known in the art to lead the gaseous sample into the ion mobility spectrometer. Preferably, the collection device comprises a device leading air, preferably air purified by methods known in the art, over the heated surface or preparation and leading the gaseous compounds together with the air into the ion mobility spectrometer. This is a preferred embodiment of sample collection since a low percentage of the signal is originating from other gaseous compounds.

In the method according to the invention an ion mobility spectrometer suitable for the determining the compounds responsible for boar taint is preferably used. The ion mobility spectrometer according to the invention has a sensitivity of 0,10 ≥ ppm skatole and 0,35 ≥ ppm androstenone, preferably 0,20 ≥ ppm skatole and 0,40 ≥ ppm androstenone and more preferably 0,25 ≥ ppm skatole and 0,50 ≥ ppm androstenone. Usable ion mobility spectrometers comprise preferably ion mobility spectrometers with an UV-ionisation source and a detection chamber based on time-domain with one Faraday detector (produced by Bruker-Saxonia, Germany and Graseby, United Kingdom) or a geometrical construction with multiple Faraday detectors (produced by Environics, Finland), but other ion mobility spectrometers known in the art can also be used.

In an embodiment of the invention the sample comprising the gaseous compounds is subjected to a separation method, preferably gas chromatography or a filtering step, before leading the sample to the ion mobility spectrometer. In this embodiment of the invention the compounds of the sample are first separated. This leads to a higher sensitivity of the present detection method. A further advantage of an additional separation step is a lower percentage of noise due to signals originating from other gaseous compounds. The separation step can comprise gas chromatographical and filtering techniques well-known in the art. Examples of usable gas chromatographical techniques are gas chromatographical techniques based on capillary columns and examples of suitable filtering techniques are filtering techniques based on molecular sieves.

The method according to invention can take place at any point along the slaughter line, but preferably at the beginning of the slaughter line so the results of the examination are available before the carcasses reach the sorting point, e.g. before being admitted to the chilling room. The method or a part of the method according to the invention can be perfromed manually or semi or fully automatically. The latter is preferred in that is it most efficient and fast. As much as 1100 examinations an hour can be preformed when a fully automatic method is used.

Furthermore, every part of the method according to the invention is preferably standardised so results of different examinations are comparable.

In a prefered embodiment of the invention the results of the method are analyzed, i.e. compared to results of meat or fat containing the unpleasant odour, i.e. boar taint, manually or by a data carrier directly after the determination of the compounds of interest. In a preferred embodiment of the invention the bodies, carcasses or parts thereof comprising the compound of interest are directly removed from the slaughter line, preferably automatically, before being admitted to the chilling room.

Furthermore, the invention covers a device for carrying out the method according to the invention. The device can be built in the slaughter line but can also be a handheld device suitable for detecting an unpleasant odour, i.e. boar taint, anywhere in the slaughter line. Optionally the device can be connected to a data carrier, comparing the results of the samples taken with a standard.

## Claims

1. A method for detecting an unpleasant odour in animal bodies, **characterized in that** said method comprises the following steps:
a) collecting a sample from an animal body;
b) determining the content of gaseous compounds in the sample by ion mobility spectrometry.

2. A method according to claim 1, wherein step a) comprises the following steps:
I) locally heating the surface of the animal body;
II) placing a sample collection device on the heated surface;
III) leading the sample in the ion mobility spectrometer.

3. A method according to claim 2, wherein the sample is subjected to gas chromatography or a filtering step before step III.

4. A method according to claim 2 or 3, wherein the heating temperature in step I is 20-120°C, preferably 30-110°C and more preferably 40-100°C.

5. A method according to any one of claims 1-4, wherein the gaseous compounds in step b) comprise skatole and androstenone.

6. A method according to claim 5, wherein the method has a sensitivity of 0,10 ≥ ppm skatole and 0,35 ≥ ppm androstenone, preferably 0,20 ≥ ppm skatole and 0,40 ≥ ppm androstenone and more preferably 0,25 ≥ ppm skatole and 0,50 ≥ ppm androstenone.

7. A method according to claim 5 or 6, further comprising the step of (c) comparing the determined skatole and androstenone content to the skatole and androstenone content of an animal body containing the unpleasant odour.

8. A method according to any of claims 1-7, wherein the method is performed in the slaughter line.

9. A method according to any of claims 1-8, wherein the unpleasant odour is boar taint.

10. A device for carrying out the method of any one of claims 1-9, comprising means for locally heating a meat surface, means for collecting a gaseous sample from a heated meat surface and an ion mobility spectrometer connected to said sample collecting device, optionally with a gas chromatograph or a filter.
